(19) Europäisches Patentamt
European Patent Office
Office européen des brevets

(11) Veröffentlichungsnummer: **0 224 871**
**A2**

(12) **EUROPÄISCHE PATENTANMELDUNG**

(21) Anmeldenummer: **86116460.6**

(51) Int. Cl.⁴: **C07D 475/14**

(22) Anmeldetag: **27.11.86**

(30) Priorität: **04.12.85 DE 3542837**

(43) Veröffentlichungstag der Anmeldung:
**10.06.87 Patentblatt 87/24**

(84) Benannte Vertragsstaaten:
**CH DE FR GB LI NL**

(71) Anmelder: **BASF Aktiengesellschaft**
**Carl-Bosch-Strasse 38**
**D-6700 Ludwigshafen(DE)**

(72) Erfinder: **Grimmer, Johannes**
**Duererstrasse 12**
**D-6700 Ludwigshafen(DE)**

(54) **Verbessertes Verfahren zur Herstellung von Riboflavin.**

(57) Verbessertes Verfahren zur Herstellung von Riboflavin der Formel I

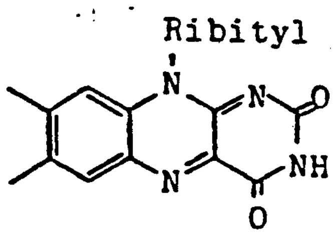

(I)

durch Kondensation eines 4,5-Dimethyl-N-(D)-ribityl-2-phenylazo-anilins der Formel II

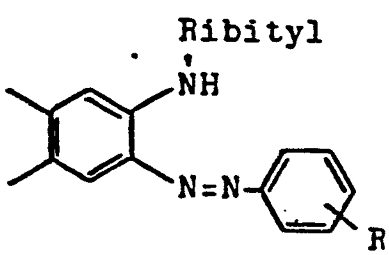

(II),

in der R H, -Cl, $-NO_2$, $-CH_3$ oder $-OCH_3$ in o-oder p-Stellung bedeutet, mit Barbitursäure der Formel III

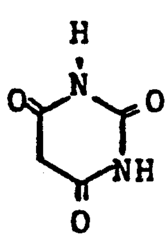

(III)

EP 0 224 871 A2

in Gegenwart einer Säure als Kondensationsmittel, das dadurch gekennzeichnet ist, daß man als saures Kondensationsmittel eine aliphatische sekundäre Carbonsäure der allgemeinen Formel IV

$$R^2-\overset{\displaystyle R^1}{\overset{|}{C}}H-COOH \qquad (IV),$$

in der $R^1$ für eine Methyl-oder Ethylgruppe und
$R^2$ für einen Alkylrest mit 3 oder 4 C-Atomen steht, verwendet.

# Verbessertes Verfahren zur Herstellung von Riboflavin

Die vorliegende Erfindung betrifft ein verbessertes Verfahren zur Herstellung von Riboflavin (I; Vitamin $B_2$) durch Kondensation eines 4,5-Dimethyl-N-(D)-ribityl-2-phenylazo-anilins (II) mit Barbitursäure (III) in Gegenwart einer Säure als Kondensationsmittel.

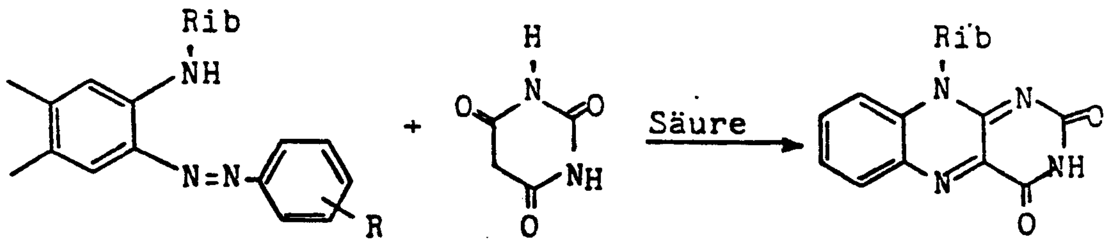


II III I

Rib = Ribityl

R = H; p-oder o-Substituent wie -Cl, $-NO_2$ oder $-CH_3$.

Dieser letzte Schritt der Riboflavin-Synthese ist, sieht man von der erfindungsgemäßen Verbesserung ab, aus mehreren Veröffentlichungen bekannt, beispielsweise aus der Arbeit von Tishler et al - (J.Am.Chem.Soc., Band 69 (1947), Seite 1487), nach welcher Eisessig als saures Kondensationsmittel verwendet wurde.

Berezowskii et al (J.Gen.Chem. USSR 1961, Seite 3444) untersuchten außerdem noch eine Reihe von anderen Säuren auf ihre Eignung als Kondensationsmittel. Hiernach erhielt man die besten Ausbeuten an I - etwa 70 % -mit Essigsäure, Phenylessigsäure und Benzoesäure. Allerdings verwendeten sie hierbei einen Barbitursäureüberschuß von 65 mol%. Setzt man II und III dagegen in äquimolaren Mengen ein, so sinkt die Ausbeute an I beträchtlich. So erhielten Haley et al (J.Am.Chem.Soc., Band 76 (1954), S. 2926) bei Verwendung von Eisessig als Kondensationsmittel bei der Umsetzung von II und III im Molverhältnis von 1:1 nur noch eine Ausbeute von 41 % an Riboflavin.

Weitaus bessere Ergebnisse wurden gemäß dem Verfahren der DE-OS 33 02 497 erzielt. Gemäß diesem Verfahren wurden als saure Kondensationsmittel aliphatische oder cycloaliphatisch-aliphatische tertiäre Carbonsäuren, insbesondere Trimethylessigsäure (Pivalinsäure) sowie geeignete handelsübliche Gemische synthetischer Säuren, die im wesentlichen gesättigte tertiäre Carbonsäuren enthalten, verwendet. Als besonders vorteilhafte Katalysatoren wurden Versatic(R)-10-Säure, eine synthetische $C_{10}$-Carbon säure der Firma Shell Chemie sowie ähnliche Produkte der Firma Esso, die unter dem Namen "Neosäuren" (z.B. Neodecansäure) im Handel sind, verwendet.

Obwohl dieses Verfahren auch technisch recht vorteilhaft durchgeführt werden kann, läßt es in einigen Punkten noch zu wünschen übrig. Zum einen sind die vorgeschlagenen Säuren, wie Versatic(R)-10-Säure, im Handel nicht unbegrenzt erhältlich und recht teuer.

Außerdem ist für die an sich besonders vorteilhaften flüssigen tertiären Carbonsäuren, wie Versatic(R)-10-Säure, die destillative Rückgewinnung verhältnismäßig aufwendig, da deren Siedebereich (90 % des Gemisches sieden unter Normaldruck bei 280°C) relativ hoch liegt und bei der destillativen Aufarbeitung des Säuregemisches nach der Umsetzung ein größerer nicht aufbereitbarer Rückstand verbleibt.

Es war daher die Aufgabe der Erfindung, einen sauren Katalysator zu finden, der technisch noch leichter zugänglich und daher noch billiger ist und der destillativ leichter rückgewinnbar ist.

Es wurde nun überraschenderweise gefunden, daß die durch ihre Verwendung bei der Herstellung von Weichmachern technisch in großem Maßstab und daher preisgünstig hergestellte 2-Ethyl-hexansäure und bestimmte Homologe davon die beschriebenen Nachteile des Standes der Technik nicht aufweisen.

Gegenstand der Erfindung ist dementsprechend ein verbessertes Verfahren zur Herstellung von Riboflavin der Formel I

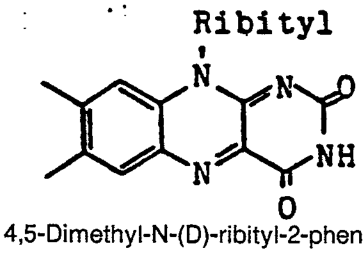

(I)

durch Kondensation eines 4,5-Dimethyl-N-(D)-ribityl-2-phenylazo-anilins der Formel II

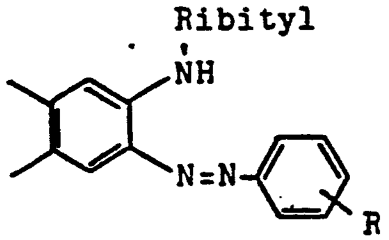

(II),

in der R H, -Cl, $-NO_2$, $-CH_3$ oder $-OCH_3$ in o-oder p-Stellung bedeutet, mit Barbitursäure der Formel III

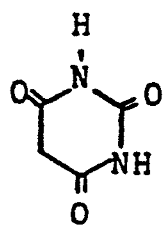

(III)

in Gegenwart einer Säure als Kondensationsmittel, das dadurch gekennzeichnet ist, daß man als saures Kondensationsmittel eine aliphatische sekundäre Carbonsäure der allgemeinen Formel IV

$$\begin{array}{c} R^1 \\ | \\ R^2\text{-}CH\text{-}COOH \end{array} \qquad (IV),$$

in der $R^1$ für Methyl oder Ethyl steht und
$R^2$ für einen Alkylrest mit 3 oder 4 C-Atomen steht,
verwendet.

Dies ist besonders überraschend, da niedere sekundäre Carbonsäuren, wie die Isobuttersäure, bei weitem nicht so gut als Katalysator einsetzbar sind. So wurde in der DE-OS 33 02 497 durch Vergleichsbeispiel 4 gezeigt, daß die Isobuttersäure als Katalysatorsäure unvorteilhaft ist.

Als aliphatische sekundäre Carbonsäuren der Formel IV kommen α-Methyl-und α-Ethyl-hexansäure sowie α-Methyl-und α-Ethylheptansäure in Betracht. Von besonderer Bedeutung ist die technisch gut zugängliche α-Ethyl-hexansäure. Sie hat einen Siedebereich von 224 bis 230°C bei 1013 mbar, der also etwa 50°C niedriger liegt als der von Versatic (R)-10-Säure.

Die Ausgangsverbindungen II sowie deren Herstellung sind bekannt. Im allgemeinen wird man die billigste Verbindung dieser Reihe, nämlich das Phenylazoderivat einsetzen. Jedoch eignen sich prinzipiell auch Verbindungen, in denen die ortho-oder insbesondere die para-Stellung der Azophenylgruppe durch Substituenten wie Methyl, Chlor, die Methoxy-oder die Nitrogruppe substituiert sind. Die Ausgangsverbindungen müssen nicht speziell gereinigt werden, sondern können auch als Rohprodukte eingesetzt werden. Die Ausbeuteangaben beziehen sich dann auf im Ausgangsmaterial enthaltenes II.

Zweckmäßigerweise nimmt man die Umsetzung, wie auch bisher schon üblich, in Gegenwart eines inerten Verdünnungs-oder Lösungsmittels vor.

Als Lösungsmittel eignen sich vorzugsweise solche, in denen auch das bei der Kondensation entstehende Wasser löslich ist oder zumindest teilweise löslich ist, also Dioxan, Tetrahydrofuran, 1-Methoxypropanol, Dimethyl-formamid, N-Methyl-pyrrolidon und vor allem die relativ billigen niederen Alkohole mit einem Siedepunkt von 80 bis 150°C, wie Propanol, Isopropanol, n-Butanol, Isobutanol und n-Pentanol, insbesondere Isobutanol.

Die Menge des Lösungsmittels soll zweckmäßigerweise so bemessen sein, daß die Ausgangsverbindungen gerade in Lösung gebracht werden können. Sie liegt im allgemeinen zwischen 2 und 12 l pro kg II. Nach kurzem Erhitzen beginnt dann das gebildete Riboflavin auszukristallisieren.

Die Menge der Carbonsäure beträgt vorzugsweise 0,5 bis 6 Mol pro Mol II. Die gemäß diesem Verfahren erzielten Ausbeuten betragen etwa 80 %, lassen sich aber noch auf über 90 % erhöhen, wenn man III in einem bis zu 0,15-molaren Überschuß verwendet. Höhere Überschüsse stören nicht, erbringen jedoch keine merklichen Ausbeutesteigerungen mehr.

Die Reaktionstemperaturen liegen bei 80 bis 120°C. Vorzugsweise arbeitet man bei Temperaturen über 100°C, also bei etwa 100 bis 115°C sowie in einem entsprechend hoch siedenden Lösungsmittel, vor allem Isobutanol. Bei 100°C beträgt die Reaktionszeit etwa 5 bis 15 Stunden.

Falls man in einem Alkohol wie Isobutanol als Lösungsmittel arbeitet, bieten die erfindungsgemäßen Säuren den Vorteil, daß sie kaum zur Veresterung neigen, so daß kaum Verluste an Lösungsmittel und Säure entstehen bzw. ein entsprechender Regenerierungsaufwand entfällt. Auch das Auswaschen der Säurereste aus dem Riboflavin gestaltet sich besonders einfach.

Die Aufarbeitung des Reaktionsgemisches kann nach den üblichen Methoden erfolgen, etwa indem man es abkühlen läßt und das auskristallisierte Riboflavin abfiltriert.

Beispiel 1 und Vergleichsbeispiele 1a und 1 b

A. Jeweils ein Gemisch, bestehend aus 40 g technischem 4,5-Dimethyl-N--(D)-ribityl-phenylazoanilin (enthaltend 85 -88 Gew.% an dem zu Riboflavin (I) cyclisierbaren 2-Phenylazo-Derivat II); entsprechend 0,096 mol), sowie aus 16 g (0,124 mol) Barbitursäure, der aus Tabelle 1 ersichtlichen Menge an Isobutanol und der aus Tabelle 1 ersichtlichen Säure wurde 10 Stunden (h) unter Rühren und Kühlung auf 108 bis 110°C zum Sieden erhitzt. Anschließend wurden die Reaktionsgemische auf 60°C abgekühlt, das auskristallisierte Roh-I abgesaugt, dann mit 200 ml an 80°C heißem Wasser und 200 ml Methanol gewaschen und dann getrocknet. In Tabelle 1 sind die Menge an gebildetem Roh-I, die sich daraus ergebende theoretische Ausbeute an Roh-I, bezogen auf enthaltenes 4,5-Dimethyl-N-(D)-ribityl-2-phenylazoanilin, sowie die pro kg eingesetzter Ausgangsverbindung erhaltene Menge an Roh-I angegeben.

B. Jeweils 20 g des gemäß A hergestellten Roh-I wurden in 200 ml Wasser suspendiert, die Suspension auf 40°C erwärmt, mit 10 g einer 25-gew.%igen wäßrigen NaOH versetzt und das Reaktionsgemisch dann 15 Minuten (Min.) gerührt. Anschließend wurde die erhaltene Lösung innerhalb von 15 Min. in ein 98 bis 100°C heißes Gemisch aus 200 ml Wasser und 15 g konz. HCl gepumpt, die erhaltene Reaktionslösung noch 1 h bei 98 bis 100°C gerührt und dann auf 40°C abgekühlt. Die erhaltenen Kristalle an Rein-I wurden dann mit 200 ml an 60°C heißem Wasser und 200 ml Methanol gewaschen. In Tabelle 1 sind die Menge an erhaltenem Rein-I, die Ausbeute an Rein-I, bezogen auf eingesetztes Roh-I, sowie die theoretischen Ausbeuten an Rein-I, bezogen auf in der Ausgangsverbindung enthaltenes 4,5-Dimethyl-N-(D)-ribityl-2-phenylazoanilin angegeben.

Die Versuche zeigen, daß 2-Ethyl-hexansäure als saurer Katalysator für die I-Herstellung weit besser geeignet ist, als die bisher technisch übliche Essigsäure und mindestens ebenso gut ist wie die gemäß dem Verfahren der DE-OS 33 02 497 bevorzugt verwendete Versatic(R)-10-Säure, der die erfindungsgemäße Säure bezüglich ihrer Zugänglichkeit und technischen Handhabbarkeit überlegen ist.

Tabelle 1

| Bsp. | Vergl. Bsp. | II [mol] | Isobutanol [ml] | Säure [ml] | Roh-Riboflavin Menge [g] | Roh-Riboflavin Ausbeuten [%, bez.auf Rein-II] | Roh-Riboflavin Ausbeuten [kg, bez.auf kg eing.II] | Rein-Riboflavin Menge [g] | Rein-Riboflavin Ausbeuten [%, bez.auf Roh-I] | Rein-Riboflavin Ausbeuten [% d. Th., bez.auf Rein-II] |
|---|---|---|---|---|---|---|---|---|---|---|
| 1 | | technisch 0,096 | 200 | Ethyl-hexans. 60 | 34,6 | 95,7 | 0,865 | 17,9 | 89,5 | 85,7 |
| | 1a | technisch 0,096 | 170 | Versatic--10-S. 100 | 33,9 | 93,8 | 0,848 | 18,2 | 91 | 85,4 |
| | 1b | technisch 0,096 | 200 | Eisessig 20 | 33,0 | 91,32 | 0,825 | 17,9 | 89,5 | 81,7 |

Beispiele 2 bis 6

Jeweils ein Gemisch, bestehend aus 40 g technischem (89-91 Gew.-%igem) 4,5-Dimethyl-N-(D)-ribityl-phenylazoanilin (enthaltend die aus der Tabelle 2 ersichtliche Mol-Menge an dem zu I cyclisierbaren 2-Phenylazo-Derivat II), sowie aus 16 g (0,124 mol) Barbitursäure, der aus Tabelle 2 ersichtlichen Menge an Isobutanol und der aus Tabelle 2 ersichtlichen Menge an 2-Ethyl-hexansäure wurde analog Beispiel 1 umgesetzt, aufgearbeitet und gereinigt.

Die erzielten Ergebnisse sind analog zu der Darstellung der Ergebnisse von Beispiel 1 (in Tabelle 1) in Tabelle 2 angegeben.

Tabelle 2

| | | | | Roh-Riboflavin | | | Rein-Riboflavin | | |
|---|---|---|---|---|---|---|---|---|---|
| | | | | | Ausbeuten | | | Ausbeuten | |
| Bsp. | II [mol] | Isobutanol [ml] | Ethyl-hexans. [ml] | Menge [g] | [%, bez.auf Rein-II] | [kg pro kg eing.II] | Menge [g] | [%, bez.auf Roh-I] | [% d. Th., bez.auf Rein-II] |
| 2 | 0,1 mol | 170 | 100 | 35,4 | 94,2 | 0,885 | 18,1 | 90,1 | 85,2 |
| 3 | 0,1 mol | 200 | 60 | 35,2 | 93,6 | 0,880 | 18,0 | 90 | 84,3 |
| 4 | 0,1 mol | 200 | 60 | 35,3 | 93,6 | 0,883 | 18,0 | 90 | 84,5 |
| 5 | 0,1 mol | 160 | 80 | 34,9 | 92,8 | 0,873 | 18,1 | 90,5 | 84,0 |
| 6 | 0,1 mol | 180 | 40 | 35,0 | 93,1 | 0,875 | 18,2 | 91 | 84,7 |

Beispiele 7 bis 10

Jeweils ein Gemisch, bestehend aus 40 g technischem 4,5-Dimethyl-N-(D)-ribityl-phenylazoanilin - (enthaltend 90 bis 91 % an dem zu I cyclisierbaren 2-Phenylazo-Derivat II; entsprechend 0,1 mol) sowie aus 16 g (0,124 mol) Barbitursäure, 80 ml 2-Ethyl-hexansäure und der aus Tabelle 3 ersichtlichen Menge des dort genannten Lösungsmittels wurde unter Rühren 10 h unter Rückfluß zum Sieden erhitzt. Nach Abkühlen auf Raumtemperatur wurde abgesaugt, das abgetrennte I mit Methanol und 80°C heißem Wasser gewaschen und dann getrocknet. Anschließend wurde das so erhaltene Roh-I analog Beispiel 1 B gereinigt. In Tabelle 3 sind die erhaltenen Mengen und theoretischen Ausbeuten (bezogen auf das in dem technischen 4,5-Dimethyl-N-(D)-ribityl-phenylazoanilin enthaltene reine 2-Phenylazo-Isomere (II)) des jeweils erhaltenen Roh-I und Rein-I angegeben.

Tabelle 3

| Bsp. | Lösungsmittel [ml] | Roh-I Menge [g] | Roh-I Ausbeute [%, bez. auf Rein-II] | Roh-I Ausbeute [kg pro kg eing. II] | Rein-I Menge [g] | Rein-I Ausbeute [%, bez. auf Roh-I] | Rein-I Ausbeute [%, bez. auf Rein-II] |
|---|---|---|---|---|---|---|---|
| 7 | Dioxan 180 | 35,3 | 93,8 | 0,88 | 18,4 | 92 | 86,3 |
| 8 | 2-Butanol 200 | 34,5 | 91,66 | 0,86 | 18,4 | 92 | 84,3 |
| 9 | Methoxypropanol 200 | 35,7 | 94,85 | 0,89 | 18,2 | 91 | 86,3 |
| 10 | Pentanol 200 | 35,2 | 93,5 | 0,88 | 18,0 | 90 | 84,2 |

Beispiel 11

Ein Gemisch, bestehend aus 20 g technischem 4,5-Dimethyl-N-(D)-ribityl-phenylazoanilin (enthaltend 90 bis 91 Gew.-% an dem zu I cyclisierbaren 2-Phenylazo-Derivat II; entsprechend 0,05 mol), sowie aus 8 g Barbitursäure, 40 ml 2-Methyl-valeriansäure und 100 ml iso-Butanol, wurde 10 h unter Rühren und Rückflußkühlung zum Sieden erhitzt. Anschließend wurde das Reaktionsgemisch auf 60°C abgekühlt, das auskristallisierte Roh-I abgesaugt und dann mit 100 ml Methanol und 100 ml an 60°C heißem Wasser gewaschen und getrocknet.

Die Ausbeute betrug 17,5 g Roh-I, entsprechend 93 % der Theorie, bezogen auf reines II.

15 g Roh-I wurde analog Beispiel I der Hochreinigung unterworfen. Man erhielt 12,7 g Rein-I, entsprechend 78,74 % der Theorie, bezogen auf reines II.

**Ansprüche**

Verbessertes Verfahren zur Herstellung von Riboflavin der Formel I

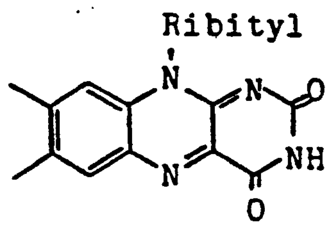

(I)

durch Kondensation eines 4,5-Dimethyl-N-(D)-ribityl-2-phenylazo-anilins der Formel II

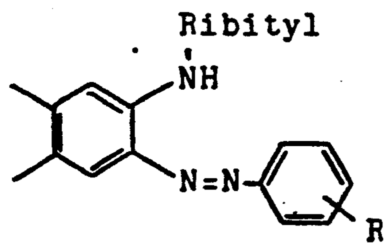

(II),

in der R H, -Cl, $-NO_2$, $-CH_3$ oder $-OCH_3$ in o-oder p-Stellung bedeutet, mit Barbitursäure der Formel III

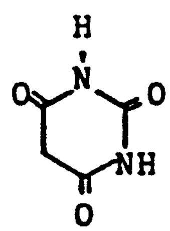

(III)

in Gegenwart einer Säure als Kondensationsmittel, dadurch gekennzeichnet, daß man als saures Kondensationsmittel eine aliphatische sekundäre Carbonsäure der allgemeinen Formel IV

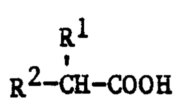

(IV),

worin $R^1$ für Methyl oder Ethyl und $R^2$ für einen Alkylrest mit 3 oder 4 C-Atomen steht, verwendet.